(19) **Europäisches Patentamt**
**European Patent Office**
**Office européen des brevets**

(11) **EP 2 997 999 A1**

(12) **EUROPEAN PATENT APPLICATION**
published in accordance with Art. 153(4) EPC

(43) Date of publication:
**23.03.2016 Bulletin 2016/12**

(51) Int Cl.:
**A61N 5/067** (2006.01)   **A61N 5/06** (2006.01)

(21) Application number: **14798487.6**

(22) Date of filing: **12.05.2014**

(86) International application number:
**PCT/JP2014/062566**

(87) International publication number:
**WO 2014/185372 (20.11.2014 Gazette 2014/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.05.2013 JP 2013101609**

(71) Applicant: **Arai Medphoton Research Laboratories, Corporation**
**Kawasaki-shi, Kanagawa 211-0063 (JP)**

(72) Inventors:
• **ARAI, Tsunenori**
  **Kawasaki-shi**
  **Kanagawa 211-0063 (JP)**

• **TAKAHASHI, Mei**
  **Yokohama-shi**
  **Kanagawa 223-8522 (JP)**
• **ITO, Arisa**
  **Kawasaki-shi**
  **Kanagawa 211-0063 (JP)**

(74) Representative: **Grünecker Patent- und Rechtsanwälte**
**PartG mbB**
**Leopoldstraße 4**
**80802 München (DE)**

(54) **THERAPY-PROGRESS-LEVEL MONITORING DEVICE AND METHOD**

(57) The present invention provides a therapy-progress-level monitoring device and a method thereof, which make it possible to know, in real time during a therapy, the therapy depth of extracellular photodynamic therapy. The device is a photodynamic therapy progress-level monitoring device (1), and is provided with: an irradiation unit (30) configured to irradiate, with excitation light of a wavelength capable of exciting a photosensitive substance, a photosensitive substance distributed and sustainably supplied by vascular permeability in the extracellular fluid and the transcellular fluid of a living body; detection unit (30, 20, 13) configured to detect, during a detection period, the fluorescence intensity produced when the photosensitive substance excited by a reaction between the excitation light and the photosensitive substance is inactivated into a ground state; a depth calculation unit (14) configured to calculate in real time an indicator value estimating the volume or the depth of a therapy region by using the fluorescence intensity during the detection period; and output unit (14, 16, 17) configured to output the indicator value in real time to a display unit or a storage unit.

FIG. 7

START
S1 CALCULATION OF FLUORESCENCE INTENSITY AT IRRADIATION START TIME
S2 Δt ELAPSES? — No
Yes
S3 CALCULATION OF FLUORESCENCE INTENSITY
S5 CALCULATION OF CORRECTED $d_{nec}$
S6 DISPLAY AND MEMORY STORAGE OF CORRECTED $d_{nec}$
S4 STEADY STATE? — No
Yes
S7 CALCULATION OF $d_{nec}$
S8 DISPLAY AND MEMORY STORAGE OF $d_{nec}$
S9 Δt ELAPSES? — No
Yes
S10 LIGHT SOURCE 11 TURNED OFF? — No
Yes
S11 CALCULATION OF FLUORESCENCE INTENSITY
S12 DATA STORAGE IN STORAGE UNIT 17
END

EP 2 997 999 A1

## Description

### TECHNICAL FIELD

The present invention is related to a therapy-progress-level monitoring device and a method thereof, for monitoring a progress level of photodynamic therapy.

### BACKGROUND ART

Tachyarrhythmia is a type of arrhythmia caused by hyperexcitability transmitted to a normal cardiac muscle tissue or formation of a turning circuit (a reentry circuit) of electric excitation in a cardiac muscle tissue. Cardiac excitation is usually controlled to a normal rate (the sinus rhythm) by excitation generated in the sinoatrial node. In the case of tachyarrhythmia, however, the heartbeat remains at a faster rate than the sinus rhythm due to hyperexcitability transmitted from some part of the cardiac tissue. The term "reentry circuit" refers to a region where electric excitation is not properly transmitted but circles in a circuit shape by the presence of a transmission damage site in the cardiac muscle tissue.

At present, a treatment for tachyarrhythmia is commonly performed by thermally damaging an abnormal site by radio frequency ablation using a catheter. Instead of using the radio frequency ablation, studies on clinical application of photodynamic therapy (PDT, also referred to as "photochemical therapy") to tachyarrhythmia have been conducted (e.g., Patent literature 1).

At present, photodynamic therapy is primarily carried out for a cancer treatment using an endoscope.

In photodynamic therapy for cancer, a photosensitive substance is administrated by a method such as intravenous injection, and after the substance is selectively absorbed and accumulated in a cancer tissue, a light beam such as a laser beam is irradiated to the cancer tissue for the treatment.

In essence, when the photosensitive substance incorporated into the cancer tissue is excited by light beam irradiation, energy from the excited photosensitive substance is transferred to oxygen present in the cancer tissue to generate active singlet oxygen, which in turn causes cell necrosis in a lesion tissue by virtue of its strong oxidizing power.

Photodynamic therapy for cancer makes use of properties of a photosensitive substance being selectively accumulated in a lesion tissue and being sensitized by light.

In photodynamic therapy for a lesion tissue such as a cancer tissue, there is proposed a device, which monitors a progress level of therapy in real time and terminates the therapy when a predetermined progress level is attained (Patent literature 2).

According to Patent literature 2, a photosensitive substance is administrated to a patient before a therapy and then light irradiation for photodynamic therapy is initiated. An amount of a photodecomposition product produced during the therapy is monitored in real time, and when the amount of the photodecomposition product produced exceeds a predetermined threshold value, a computer closes a shutter of laser to terminate photodynamic therapy.

According to the device in Patent literature 2, it is convenient that a medical doctor is informed of the progress level of therapy in real time during the operation as well as the completing time of the therapy in an automatic mode.

### CITATION LIST

### PATENT DOCUMENT

Patent Document 1: WO 2008/ 066206
Patent Document 2: U.S. Pat. No. 6,128,525

### DISCLOSURE OF THE INVENTION

Problem to be Solved by the Invention

Although, by using the device in Patent literature 2, it was possible to monitor the progress level of photodynamic therapy for a cancer tissue and the like, where the therapy utilizes accumulation property of a photosensitive substance in a lesion tissue, it was not possible to monitor the progress level of photodynamic therapy for infectious diseases, arrhythmia, and the like, where the therapy is performed in a situation that requires a sufficient amount of a photosensitive substance to be distributed and sustainably supplied in the extracellular interstitium and the blood vessel in a therapy-target tissue.

That is, since photodynamic therapy for a cancer tissue and the like utilizes the accumulation property of a photosensitive substance in a lesion tissue, it is possible to estimate an amount of a photosensitive substance to be consumed and an accurate amount of a photodecomposition product to be produced during the therapy by examining a lesion before starring the therapy.

In contrast, photodynamic therapy for infectious diseases and arrhythmia, instead of utilizing the accumulation property of a photosensitive substance, is performed in a situation where a sufficient amount of a photosensitive substance is distributed and sustainably supplied in the extracellular interstitium and the blood vessel in a therapy-target tissue.

In particular, in photodynamic therapy for arrhythmia, an electrophysiological examination is performed to locate a transmission route of an electric signal and the therapy is performed to disrupt the route instantly while being located. This operation is carried out under a fully monitored situation while the electrophysiological examination is performed, and an instant evaluation needs to be made on the therapy. Thus, it is necessary to achieve an electrical conduction block in the cardiac muscle tissue by performing the therapy instantly.

This makes it impossible to predict the accurate amount of the photodecomposition product produced during the therapy before the therapy, thus it is impossible for the device in Patent literature 2 to estimate the progress level of therapy in real time during the therapy.

Further, although it is possible to determine whether an electrical conduction block is achieved or not by electrocardiogram, there is no means available to a practitioner for knowing in real time a level of the depth of damages continuously caused to a tissue by light radiation from a laser. This has been a barrier of clinical application of photodynamic therapy for treating arrhythmia.

Up to now, a device and a method, capable of monitoring a therapy depth in real time during a therapy, have not been known in photodynamic therapy for infectious diseases and arrhythmia, where sufficient amounts of a photosensitive substance and oxygen are always supplied by the bloodstream and a sufficient amount of light is always supplied by light irradiation.

The present invention has been completed based on the knowledge resulting from intensive studies conducted by the inventors in view of the above-mentioned circumstances and an object of the present invention is to provide a therapy-progress-level monitoring device and a method thereof, for allowing a user to know a therapy depth in real time during a therapy in extracellular photodynamic therapy (Extracellular PDT), where a photosensitive substance is distributed extracellularly and a photosensitizing reaction occurs extracellularly.

Means for Solving Problem

The present inventors have conducted extensive studies on a method for monitoring a therapy depth in extracellular photodynamic therapy. As a result, the inventors found that, in a situation where a photosensitive substance is distributed and sustainably supplied by vascular permeability in an extracellular fluid such as the blood plasma and the interstitial fluid and/or a transcellular fluid such as the cerebrospinal fluid, the gastrointestinal tract inner fluid, the pericardial inner fluid, the joint fluid, and the ocular fluid in a living body, there is a correlation between fluorescence intensity produced when the photosensitive substance is inactivated to a ground state after having being excited by excitation light, and a volume or a depth of a therapy region of photodynamic therapy. The present invention has thus been accomplished based on such knowledge.

According to a therapy-progress-level monitoring device of an embodiment, the above problem is solved by the device that evaluates a progress level of photodynamic therapy for treating a therapy region of a living body by using a photosensitive substance excited by light having a peak intensity in a predetermined range, the device comprising: an irradiation unit configured to irradiate, with excitation light of a wavelength capable of exciting the photosensitive substance, the photosensitive substance distributed and sustainably supplied by vascular permeability in an extracellular fluid and/or a transcellular fluid of the living body; a detection unit configured to detect, during a detection period, fluorescence intensity produced when the photosensitive substance excited by a reaction between the excitation light and the photosensitive substance is inactivated into a ground state; a depth calculation unit configured to calculate in real time an indicator value estimating a volume or a depth of the therapy region by using the fluorescence intensity during the detection period, received from the detection unit; and an output unit configured to output in real time the indicator value calculated by the depth calculation unit to a display unit or a storage unit.

According to a therapy-progress-level monitoring device of an embodiment, the above problem is solved by the device that evaluates a progress level of photodynamic therapy for treating a therapy region of a living body by using a photosensitive substance excited by light having a peak intensity in a predetermined range, the device comprising: a receiving unit configured to receive a value of fluorescence intensity detected by a detection unit configured to detect, during a detection period, the fluorescence intensity produced when the photosensitive substance excited by a reaction between excitation light and the photosensitive substance is inactivated into a ground state, wherein the excitation light has a wavelength capable of exciting the photosensitive substance and is irradiated to the photosensitive substance distributed and sustainably supplied by vascular permeability in an extracellular fluid and/or a transcellular fluid of the living body; a depth calculation unit configured to calculate in real time an indicator value estimating a volume or a depth of the therapy region by using the received value of the fluorescence intensity during the detection period; and an output unit configured to output the indicator value calculated by the depth calculation unit to a display unit or a storage unit for allowing real time displaying and storing.

According to a therapy-progress-level monitoring method of an embodiment, the above problem is solved by the method that evaluates a progress level of photodynamic therapy for treating a therapy region of a living body by using a photosensitive substance excited by light having a peak intensity in a predetermined range, the method comprising: a receiving procedure configured to receive a value of fluorescence intensity detected by a detection unit configured to detect, at a predetermined time point during a detection period, the fluorescence intensity produced when the photosensitive substance excited by a reaction between excitation light and the photosensitive substance is inactivated into a ground state, wherein the excitation light has a wavelength capable of exciting the photosensitive substance and is irradiated to the photosensitive substance distributed and sustainably supplied by vascular permeability in an extracellular fluid and/or a transcellular fluid of the living body; a depth calculation procedure configured to calculate in real time an indicator value estimating a volume or a depth of the therapy region by using the received value of the fluorescence intensity at the predetermined time point; and an output procedure configured to output the indicator value calculated by the depth calculation procedure to a display unit or a storage unit for allowing real time displaying and storing.

Since the present invention has such a configuration, in photodynamic therapy that does not utilize the accumulation property of a photosensitive substance, such as the one for infectious diseases and arrhythmia, a practitioner can easily know, in real time during the therapy, whether the therapy has been progressed to an intended level or not, or when the therapy should be terminated.

Further, according to the present invention, it becomes possible to monitor a progress level of a therapy in real time during the therapy in photodynamic therapy in a situation where a photosensitive substance is distributed and sustainably supplied by vascular permeability in an extracellular fluid and/or a transcellular fluid of a living body, thus the present invention paves the way for realizing photodynamic therapy for arrhythmia.

In this configuration, the detection unit may further detect intensity of light having a wavelength of the excitation light irradiated by the irradiation unit as return excitation light intensity, and the depth calculation unit may calculate the indicator value using absolute values or relative values of the return excitation light intensity during the detection period, received from the detection unit, and the fluorescence intensity.

As above, the depth calculation unit calculates the indicator value using not only the fluorescence intensity, but also the return excitation light intensity during the detection period, received from the detection unit, thus a progress level of therapy, which has correlation with excitation light intensity, can be evaluated more accurately. Further, the depth calculation unit uses the absolute values or the relative values of the light intensity to calculate the indicator value, thus a progress level of therapy can be evaluated more accurately.

In this configuration, the detection period may be a time period from a first light emission period from an irradiation start time of the excitation light until when the fluorescent intensity reaches a steady state to a second light emission period having the steady state continued from the first light emission period until when irradiation of the excitation light is ended, or a time period including only the second light emission period.

Since the present invention has such a configuration, in extracellular photodynamic therapy, it is possible to monitor a progress level of therapy at least in the second light emission period during which a photosensitive substance is used primarily for tissue damages, following the first light emission period during which photodynamic therapy causes significantly more damages to a serum protein associated with a photosensitive substance as compared to tissue damages.

In this configuration, the irradiation unit may comprise a laser catheter or a optical fiber and the depth calculation unit may calculate a PBI (photosensitizer bleaching index), serving as a progress indicator of photodynamic therapy, by multiplying a difference between the fluorescence intensity at an irradiation start time of the excitation light and the fluorescence intensity at a specific time of irradiation by a time from the irradiation start time to the specific time of irradiation, and then calculate an indicator value estimating a volume or a depth of the therapy region using the PBI, wherein the specific time of irradiation may be selected from a time range from the irradiation start time of the excitation light to a time when a contact state of a tip portion of the laser catheter or the optical fiber to a tissue of the living body changes.

As above, the depth calculation unit is configured to calculate a PBI (photosensitizer bleaching index), serving as a progress indicator of photodynamic therapy, by multiplying a difference between the fluorescence intensity at an irradiation start time of the excitation light and the fluorescence intensity at a specific time of irradiation by a time from the irradiation start time to the specific time of irradiation, and then calculate an indicator value estimating a volume or a depth of the therapy region using the PBI, thus the indicator value estimating the volume or the depth of the therapy can be a highly useful value matching to a actual state.

Further, the specific time of irradiation is selected from a time range from the irradiation start time of the excitation light to a time when a contact state of a tip portion of the laser catheter or the optical fiber to a tissue of the living body changes, thus the indicator value can be obtained at least from the irradiation start time of the excitation light to the time when a state of the tissue of the living body changes.

A time point when a state of the tissue of the living body changes can be interpreted that the therapy is drawing to its end, thus by obtaining the indicator value from the irradiation start time of the excitation light to the time when a state of the tissue of the living body changes, a whole period of the therapy can be monitored.

In this configuration, the depth calculation unit may comprise a correction unit configured to correct the indicator value, wherein the correction unit executes at least one processing selected from a group consisting of: a processing of subtracting the PBI in a final stage of the first light emission period from the PBI; a processing of correcting the PBI in accordance with a state in progress in order for the PBI to reflect a photosensitizing reaction in a transition state of the first light emission period using ratios between the fluorescence intensity in the first light emission period and at the irradiation start time of the excitation light, and the fluorescence intensity in the second light emission period, when the specific time of irradiation is any time during the first light emission period; a processing of multiplying the PBI by a detection value of the fluorescence intensity at the irradiation start time of the excitation light; a processing of multiplying the PBI by a ration of the fluorescence intensity in the second light emission period to the fluorescence intensity at the irradiation start time of the excitation light; a processing of dividing the PBI by the return excitation light intensity at the specific time of irradiation; and a processing of correcting the PBI by using physiological information on a tissue obtained through measurement by other methods, information on systemic conditions of the living body obtained from other living-body monitoring devices, and known physiological numerical values related to a tissue according to the therapy region, to calculate a corrected PBI, the depth calculation unit may calculate an indicator value estimating a volume or a depth of the therapy region using the corrected PBI in real time or after the first light emission period, and the output unit may output the indicator value calculated by the depth calculation unit to a display unit or a storage unit in real time or after the first light emission period.

By subtracting the PBI in a final stage of the first light emission period from the PBI by the correction unit, a progress level of therapy can be more accurately estimated with consideration of a photosensitizing reaction in a transition state of the first light emission period, in which photodynamic therapy causes significantly more damages to a serum protein associated with a photosensitive substance as compared to tissue damages.

Further, by correcting the PBI in accordance with a state in progress in order for the PBI to reflect a photosensitizing reaction in a transition state of the first light emission period using ratios between the fluorescence intensity in the first light emission period and at the irradiation start time of the excitation light, and the fluorescence intensity in the second light emission period, when the specific time of irradiation is any time during the first light emission period, it becomes possible to obtain a corrected PBI, which reflect an actual state of the first light emission period being different from the steady state of the second light emission period in a balance between supply of a PDT agent and oxygen by vascular permeability or the bloodstream and consumption of a PDT agent and oxygen by photodynamic therapy.

Further, by multiplying the PBI by a detection value of the fluorescence intensity at the irradiation start time of the excitation light by the correction unit, a progress level of therapy can be more accurately estimated by including a concentration of a photosensitive substance distributed in a tissue.

By the processing of multiplying the PBI by a ration of the fluorescence intensity in the second light emission period to the fluorescence intensity at the irradiation start time of the excitation light or the processing of dividing the PBI by the return excitation light intensity at the specific time of irradiation, performed by the correction unit, a progress level of therapy can be more accurately estimated by including differences in the optical characteristics among target tissues for therapy.

By the processing of correcting the PBI by using physiological information on a tissue obtained through measurement by other methods, information on systemic conditions of the living body obtained from other living-body monitoring devices, and known physiological numerical values related to a tissue according to the therapy region, performed by the correction unit, a progress level of therapy can be more accurately estimated by including differences in the physiological characteristics among target tissues for therapy.

In this configuration, the depth calculation unit may calculate an indicator value estimating a volume or a depth of the therapy region expressed as a relative value using a logarithm of the PBI or the corrected PBI, and the output unit may output the indicator value expressed as a relative value to the display unit or the storage unit.

With this configuration, the indicator value estimating the volume or the depth of the therapy region is not displayed as an absolute value in the display unit during the therapy, allowing a practitioner to have room for evaluating a progress level of therapy.

Further, intensive studies conducted by the inventors have proven that there is a correlation in a logarithmic scale between a PBI and a measured depth of necrosis in a so-called extracellular photodynamic therapy according to the present invention, thus, it becomes possible to estimate a volume or a depth of a therapy region using a logarithm of a PBI or a logarithm a corrected PBI.

In this configuration, the photosensitive substance may be talaporfin sodium.

In this configuration, the excitation light may be light having a wavelength of 400 to 700nm and may be light emitted from a laser beam, a light emitting diode, or a lump source.

In this configuration, the detection unit may include a detection element selected from a group consisting of a silicon photodiode, an avalanche photodiode, a spectrometer, and a photomultiplier tube, and detect the fluorescence and the return excitation light at a sampling rate of 10 to 1,000Hz.

ADVANTAGEOUS EFFECTS OF INVENTION

According to the present invention, in photodynamic therapy that does not utilize the accumulation property of a photosensitive substance, such as the one for infectious diseases and arrhythmia, a practitioner can easily know in real time during the therapy whether the therapy has been progressed to an intended level or not, or when the therapy should be terminated.

Further, according to the present invention, it becomes possible to monitor a progress level of therapy in real time during the therapy in photodynamic therapy conducted in a situation where a photosensitive substance is distributed and sustainably supplied by vascular permeability in an extracellular fluid and/or a transcellular fluid of a living body, thus the present invention paves the way for realizing photodynamic therapy for arrhythmia.

BRIEF DESCRIPTION OF DRAWINGS

FIG. 1 is a schematic explanatory diagram illustrating a principle and a reaction mechanism of photodynamic therapy for arrhythmia, in which a therapy-progress-level monitoring device according to one embodiment of the present invention is used.

FIG. 2 is an explanatory diagram illustrating a reaction dynamics of extracellular photodynamic therapy when a therapy-progress-level monitoring method according to one embodiment of the present invention is carried out.

FIG. 3 is an explanatory diagram illustrating a concept of a PBI.

FIG. 4 is a block diagram illustrating an approximate configuration of the therapy-progress-level monitoring device according to one embodiment of the present invention.

FIG. 5 is a schematic diagram illustrating a measurement of fluorescence and return excitation light when excitation light is irradiated from a laser catheter to a therapy-target tissue.

FIG. 6 is a graph showing a time-dependent change of fluorescence detector outputs in extracellular photodynamic therapy in Example of the present invention.

FIG. 7 is a flowchart illustrating the processing of the therapy-progress-level monitoring method according to one embodiment of the present invention.

FIG. 8 is a photograph showing a measured depth of necrosis $D_{nec}$ in a tissue treated by extracellular photodynamic therapy in Example of the present invention.

FIG. 9 is a graph showing a correlation between PBI calculated values and the measured depth of necrosis $D_{nec}$, in the tissue treated by extracellular photodynamic therapy in Example of the present invention.

DETAILED DESCRIPTION OF THE INVENTION

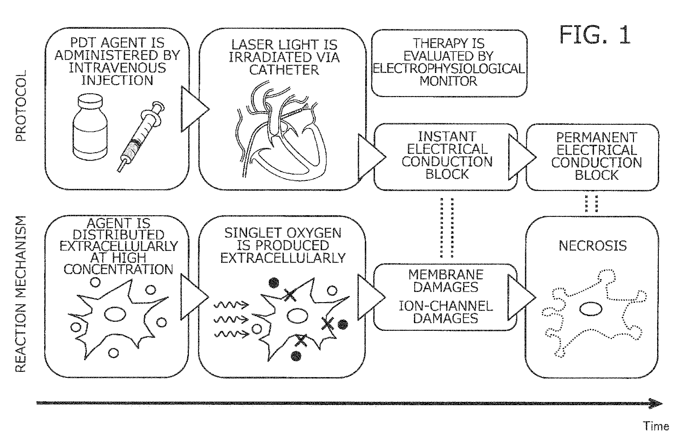

Hereinafter, a therapy-progress-level monitoring device according to one embodiment of the present invention will be described with reference to Fig. 1 to Fig. 9.

A therapy-progress-level monitoring device 1 of the present embodiment is a device that calculates and displays on a screen an indicator value of a therapy depth in real time during a treatment of extracellular photodynamic therapy (Extracellular PDT), where a sufficient amount of a photosensitive substance (hereinafter referred to as a PDT agent) is distributed in the extracellular interstitial fluid and in the blood in a therapy-target tissue. A practitioner can estimate a depth of damages of the therapy-target tissue caused by light irradiation at every moment while referring to the indicator value of the therapy depth being displayed on the screen in real time during the therapy.

The extracellular photodynamic therapy of the present embodiment is performed in a situation where the PDT agent is distributed and sustainably supplied by vascular permeability in an extracellular region of a living body, i.e., in an extracellular fluid and/or a transcellular fluid.

In the present embodiment, the therapy-progress-level monitoring device 1 is used for treating arrhythmia, however it is also suitable for extracellular photodynamic therapy, where a sufficient amount of a PDT agent is distributed in the extracellular interstitial fluid and in the blood in a therapy-target tissue, thus it can be also used for other therapy, such as photodynamic therapy for infectious diseases. The device can be used for cancer treatments (including gastrointestinal division, respiratory division, brain surgery, and dermatology) in which a PDT agent is accumulated intracellularly in a therapy-target tissue, arteriosclerosis treatments, and the like, as long as the sufficient amounts of the PDT agent and oxygen are sustainably supplied to the extracellular interstitial fluid and in the blood in the therapy-target tissue. The device may be also used for angioplasty.

Arrhythmia to be treated by using the therapy-progress-level monitoring device 1 of the present embodiment is a type of arrhythmia caused by the presence of an abnormal electrical conduction site or a site at which hyperexcitability occurs, and in particular, tachyarrhythmia including all types of tachyarrhythmia, which have been conventionally treated by radio frequency ablation. Examples of tachyarrhythmia include: atrial fibrillation (AF) such as paroxysmal AF, persistent AF, and permanent AF; atrial flutter (AFL); and paroxysmal supraventricular tachycardia such as atrioventricular reciprocating tachycardia (AVRT), atrioventricular nodal reentrant tachycardia (AVNRT), and atrial tachycardia (AT).

Further, examples of infectious diseases include MRSA infection, gingivitis, periodontitis, peri-implant diseases, herpes, oral ulcer, candidiasis, and the like.

A PDT agent used in the present embodiment is a photosensitive substance.

In the present embodiment, unlike photodynamic therapy for cancer, it is not necessarily to accumulate an agent in a tissue, thus a treatment is started shortly after the administration of the agent by setting Drug Light interval from administration of agent to light irradiation to a short time, about several minutes to several tens of minutes. Accordingly, it is preferred to use a water-soluble photosensitive substance, which is rapidly distributed in the interstitium after the administration by intravenous injection and the like, and rapidly discharged.

Examples of such an agent include a chlorine-based agent having a chlorine skeleton, such as ATX-S10 (670nm) (iminochlorin aspartic acid derivative, Oriental Menthol Industry Ltd., rights were transferred to Photochemical Co., Ltd. in 2000, JP-A-6-80671), NPe6 (664nm) (talaporfin sodium, Laserphyrin (registered trademark), mono-L-aspartyl chlorine 6, JP Pat. No. 2961074), mTHPC (652nm), SnET2 (660nm) (tin etiopurpurin, Miravant Medical Technologies), AlPcS (675nm) (chloroaluminium sulphonated phthalocyanine), BPD-MA (690nm) (benzoporphyrin derivative monoacid ring A, QLT Inc.), Lu-tex (732nm) (Lutetium Texaphyrin), and the like. Of these, talaporfin sodium is preferable.

«Principle and reaction mechanism of extracellular photodynamic therapy in therapy-progress-level monitoring method»

Based on Fig. 1, explanation is given for a principle and a reaction mechanism of photodynamic therapy for arrhythmia, in which a therapy-progress-level monitoring device 1 according to the present embodiment is used.

In photodynamic therapy for arrhythmia according to the present embodiment, the therapy utilizes a photosensitizing reaction occurring in the early stage after the administration of a PDT agent, i.e., in a timing that the PDT agent is distributed extracellularly (in an interstitial space) instead of intracellularly.

After the PDT agent is administered by intravenous injection and the like, the agent is initially distributed in the extracellular interstitium and in the blood vessel at a high concentration. During this period, an extracellular region is in a state of being sustainably supplied with the PDT agent and oxygen at a sufficient level by the bloodstream. The PDT agent is supplied to the interstitium from the blood vessel by permeability.

When laser light is irradiated to a target site via a catheter by setting Drug Light interval from administration of agent to light irradiation to a short time, about several minutes to several tens of minutes, light is supplied at a sufficient level by the laser light while the PDT agent and oxygen is supplied at a sufficient level by the bloodstream. As a result, a photosensitizing reaction by the PDT agent, light, and oxygen occurs.

In the photosensitizing reaction, the PDT agent is excited by light beam irradiation. Energy of the excited PDT agent is transferred to oxygen in the extracellular region to generate active singlet oxygen (active oxygen). By virtue of a strong oxidizing power of the singlet oxygen, as an effect of the therapy, the disappearance of electric conductivity in a cardiac muscle cell due to oxidation damages to ion channels and cell membranes thereof as well as necrosis are achieved.

In essence, photodynamic therapy requires a PDT agent, excitation light that excites the PDT agent, and oxygen that is activated by the excited PDT agent to be in an extracellular region, and these three factors are extremely important for a therapy that produces singlet oxygen to cause necrosis.

While selectivity in photodynamic therapy for cancer is dependent on selective accumulation of a PDT agent to a lesion, selectivity in photodynamic therapy according to the present embodiment is controlled by light irradiation.

In extracellular photodynamic therapy, a PDT agent is distributed in the interstitium or in the blood vessel, thus the PDT agent exists in a bounded state to a serum protein such as albumin, high-density lipoprotein (HDL), and low-density lipoprotein (LDL).

Singlet oxygen produced by a protein-bound PDT agent is consumed by oxidation of the bound protein. It is singlet oxygen derived from a PDT agent not bound to a protein that exerts a therapeutic effect.

Thus, when photodynamic therapy is started, singlet oxygen is consumed by oxidation of a bound protein immediately after starting, subsequently a detection value of fluorescence is shifted to a steady state being substantially constant and a therapeutic effect is exerted.

A first light emission period of the present specification may correspond to a period when oxidation of a bound protein by singlet oxygen occurs. A second light emission period may correspond to a period after oxidation of a bound protein by singlet oxygen is finished.

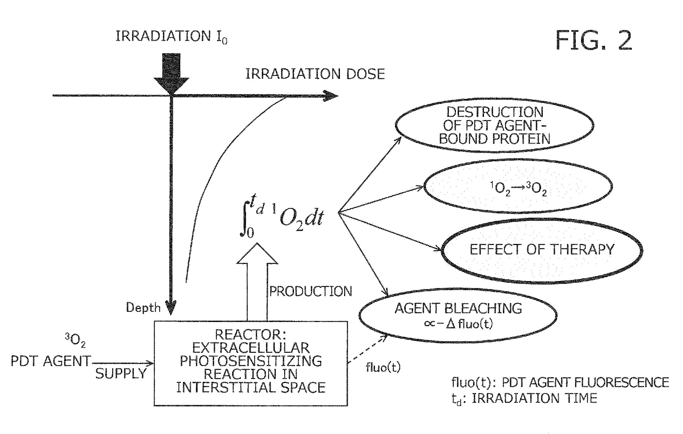

FIG. 2 shows a reaction dynamics of extracellular photodynamic therapy when the therapy-progress-level monitoring method is performed using the therapy-progress-level monitoring device 1 according to the present embodiment.

When red light is irradiated in the presence of triplet ground-state oxygen and a PDT agent, excited singlet oxygen is produced by a photosensitizing reaction occurring in an extracellular interstitial space. A total production amount of singlet oxygen is an irradiation-time integration value of an amount of singlet oxygen per unit time.

When the excited singlet oxygen is produced, a protein bound to the PDT agent is destroyed, the excited singlet oxygen is converted to triplet ground-state oxygen, and agent bleaching occurs. As a result, a therapeutic effect is obtained.

The term "agent bleaching" described here is a phenomenon that a photosensitive substance serving as a PDT agent is destroyed by singlet oxygen. A quantity of agent bleaching is proportional to a changing amount of PDT agent fluorescence per unit time.

<<PBI (Photosensitizer bleaching index)>>

In the present embodiment, a PBI (Photosensitizer bleaching index) is used as an indicator value of a therapy depth of extracellular photodynamic therapy.

In an extracellular photosensitizing reaction occurring in extracellular photodynamic therapy, a PDT agent is sufficiently supplied by the bloodstream during the therapy, thus it is considered that time integration of a value of agent bleaching corresponds to a total amount of agent bleaching up to that time point, i.e., a total production amount of singlet oxygen, serving as a therapeutic effect factor.

Therefore, in the present embodiment, as an indicator corresponding to the total amount of the bleaching, a PBI represented by the following formula I is used.

[Mathematical formula 1]

$$PBI = (fluo(0) - fluo(t_d))t_d \qquad \text{(formula 1)}$$

It is noted that, in formula 1, the following abbreviations are used:

$fluo(t)$: return fluorescence intensity at a time point t

$t_d$: light irradiation time [s]

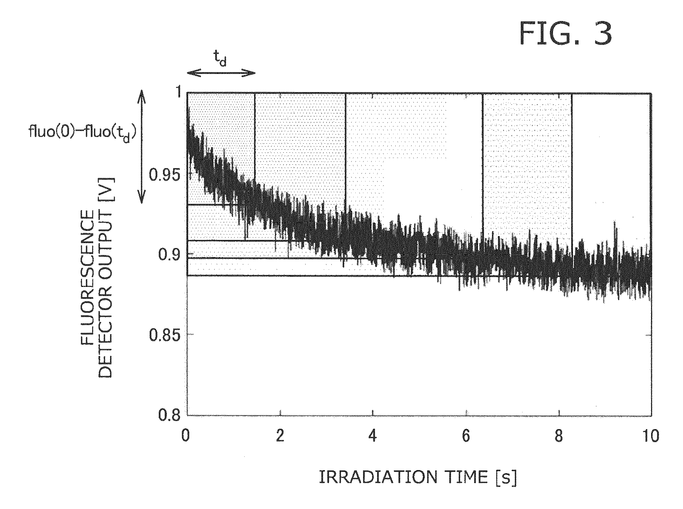

A concept of the PBI is explained based on Fig. 3. Fig. 3 is a graph showing a time-dependent change of return fluorescence intensity in an extracellular photosensitizing reaction, where a plurality of rectangles, representing a concept of the PBI, are drawn in superposition.

The PBI is a value obtained by multiplying a difference obtained by subtracting fluorescence intensity at a start time of light irradiation to from fluorescence intensity at a light irradiation time $t_d$ indicating a measurement time by the light irradiation time $t_d$ up to the measurement time.

In Fig. 3, sides of the plurality of rectangles in a lateral axis direction correspond to the irradiation time $t_d$ up to the measurement time.

Further, at the start time of light irradiation to, i.e., at a time 0 in Fig. 3, light irradiation is not initiated yet, thus the agent bleaching doesn't occur and return fluorescence intensity $fluo(t)$ shows the highest value. Subsequently, after a time 0, light irradiation is performed, and as the measurement time $t_d$ passes, the agent bleaching proceeds and the $fluo(t)$ decreases.

Thus, sides of the plurality of rectangles in a longitudinal axis direction correspond to a difference obtained by subtracting the fluorescence intensity at the start time of light irradiation to from the fluorescence intensity at the light irradiation time $t_d$ indicating the measurement time.

Consequently, the PBI corresponds to an area of each rectangle in Fig. 3.

In formula 1, it is assumed that oxygen and a PDT agent is sufficiently supplied at a constant rate by permeability from the blood vessel to the interstitium. Thus, a rate-determining step is an amount of light energy to be charged.

Further, measurement values of fluorescence include measurement values of fluorescence both from the blood and the interstitium, wherein an abundance ratio of the fluorescence therebetween is assumed to be constant.

In extracellular photodynamic therapy, it is assumed that a singlet oxygen production amount is proportional to an amount of light energy to be charged, thus a estimated value of necrosis depth by an extracellular photosensitizing reaction, i.e., an estimated therapy depth $d_{nec}$, depends on light distribution in a depth direction.

Accordingly, the estimated therapy depth $d_{nec}$ can be expressed as a logarithm of the PBI in the following formula 2, obtained by modifying a general formula indicating an absorption coefficient, $E_{(d)} = I_0 t \cdot \exp(-\mu_{eff}d)$.

[Mathematical formula 2]

$$d_{nec} = \frac{1}{\mu_{eff}} \ln\left(\frac{k(t)I_0 PBI}{E}\right)$$

$$= \frac{1}{\mu_{eff}} \left\{ \ln(PBI) + \ln\left(\frac{k(t)I_0}{E}\right) \right\} \qquad \text{(formula 2)}$$

It is noted that, in formula 2, the following abbreviations are used:

$d_{nec}$: damage depth [mm]
$\mu_{eff}$: attenuation coefficient [/mm]
$k(t)$: constant that varies with time $1/\{fluo(0)-fluo(t)\}$
$I_0$: light intensity on tissue surface [mW]
$E$: light energy at given depth [J]

<<Correction of PBI>>

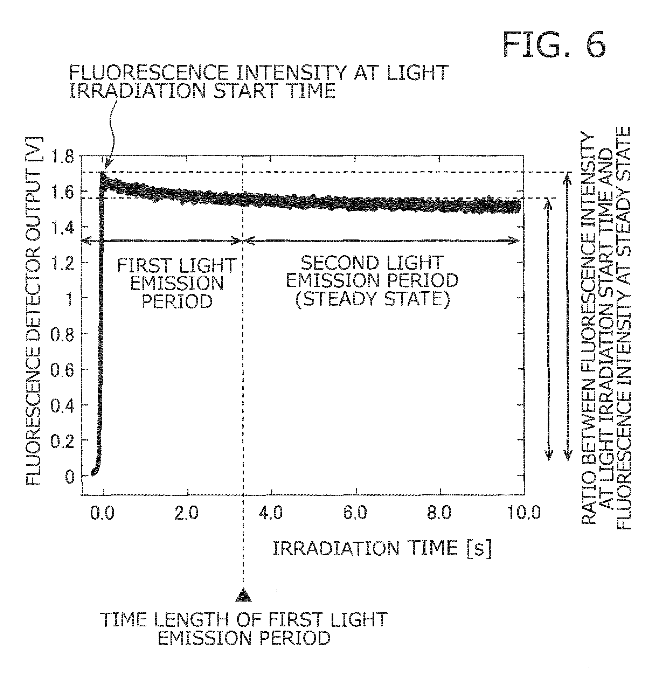

The PBI obtained by formula 1 can be used to provide a highly useful indicator matching to an actual state by calculating a corrected PBI that corrects the PBI.
Methods for correction can be divided into correction methods that utilize a temporally changing fluorescence intensity waveform itself and correction methods that utilize physiological information on a tissue and the like obtained by other methods.
Among the correction methods that utilize a temporally changing fluorescence intensity waveform itself, the following 1, 2 and 3 are explained based on a graph in Fig. 6. FIG. 6 is a graph showing a time-dependent change of fluorescence detector outputs in extracellular photodynamic therapy, obtained in Example mentioned below.
As shown in Fig. 6, it is confirmed that, in extracellular photodynamic therapy, fluorescence intensity detected by a fluorescence detection unit 12 reaches a steady state some time after a start of light irradiation.
Thus, a time period from the start of light irradiation to the end of light irradiation can be divided into a first light emission period from the start of light irradiation until when fluorescent intensity reaches a steady state and a second light emission period having the steady state continued from the first light emission period until the end of light irradiation.

Correction method 1

A corrected PBI can be obtained by multiplying the PBI obtained by formula 1 by a fluorescence intensity output at the start time of light irradiation.
The fluorescence intensity at the start time of light irradiation corresponds to a concentration of a PDT agent, thus such correction makes it possible to obtain a corrected PBI, which includes a concentration of a PDT agent distributed in a tissue.

Correction method 2

A ratio between the fluorescence intensity at the start time of light irradiation and the fluorescence intensity at a steady state indicates a transition state in the first light emission period proceeding to the steady state.
A PBI indicates a reaction progress in the steady state. Thus, the PBI is corrected in accordance with a situation in progress by using two indicators, namely a fluorescence intensity ratio between in the first light emission period and in the steady state and a fluorescence intensity ratio between at the start time of light irradiation and in the steady state, so as to reflect a photosensitizing reaction occurring in the transition state in the first light emission period.
The first light emission period is different from the second light emission period having the steady state in a balance between supply of a PDT agent and oxygen by vascular permeability or the bloodstream and consumption of a PDT agent and oxygen by photodynamic therapy.
Thus, according to the correction method 2, there can be obtained a corrected PBI that matches to an actual state of the first light emission period having the transition state, which is caused by being different from the steady state in a balance between supply of a PDT agent and oxygen by vascular permeability or the bloodstream and consumption of a PDT agent and oxygen by photodynamic therapy.

Correction method 3

A corrected PBI can be obtained by dividing the PBI obtained by formula 1 by return excitation light intensity at an irradiation time in progress.

With this correction, there can be obtained the corrected PBI that includes differences in the optical characteristics among target tissues for therapy.

In the correction methods that utilize physiological information on a tissue and the like obtained by other methods, a correction is performed by using physiological information on a tissue obtained through measurement by other methods, information on systemic conditions of a patient obtained by general living-body monitoring devices, or known physiological numerical values related to a target tissue.

Examples of the physiological information, the information on systemic conditions, and the physiological numerical values include oxygen concentrations such as $PaO_2$ (partial pressure of oxygen in arterial blood) and SpO2 (endermic arterial oxygen saturation), hemoglobin contents such as a total hemoglobin content, an oxygenated hemoglobin content, and a deoxygenated hemoglobin content, a blood flow rate in each tissue, a bloodstream quantity, a vessel running density per unit volume, a total blood volume, patient's temperature, temperature of a target tissue, and concentrations of serum proteins such as albumin, high-density lipoprotein (HDL), and low-density lipoprotein (LDL).

Of those, the oxygen concentrations are factors that affect reaction efficiency and oxygen supply. Further, the hemoglobin contents are factors that affect oxygen supply. The bloodstream quantity and the blood flow rate are factors that affect supply of a PDT agent and oxygen. The temperatures are a factor that affects reaction efficiency. The serum protein contents affect a supply quantity of a PDT agent because a serum protein binds to a PDT agent.

«Configuration of therapy-progress-level monitoring device 1»

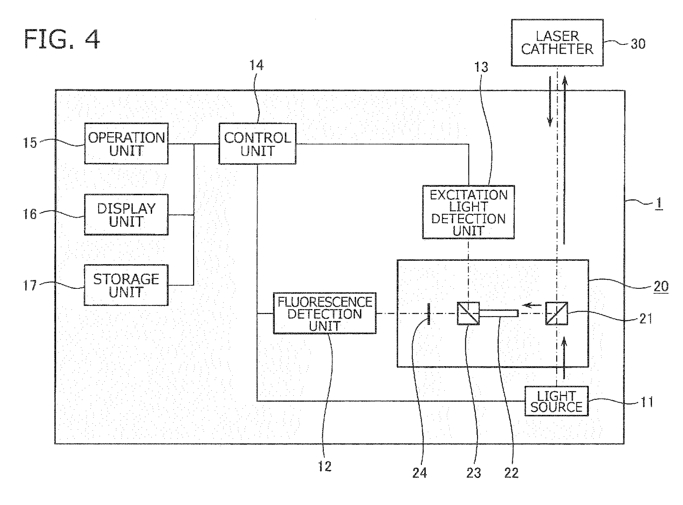

Next, a therapy-progress-level monitoring device 1 of the present embodiment is explained based on Fig. 4.

FIG. 4 is a block diagram illustrating an approximate configuration of the therapy-progress-level monitoring device I according to the present embodiment.

The therapy-progress-level monitoring device 1 includes a light source 11, an optical system 20, a fluorescence detection unit 12, a return excitation light detection unit 13, a control unit 14, an operation unit 15, a display unit 16, and a storage unit 17.

The light source 11 outputs excitation light that excites a PDT agent. A wavelength of the light output by the light source 11 is the same as a Q-band absorption wavelength of the PDT agent. Talaporfin sodium is used as a PDT agent in the present embodiment, thus as the light source 11, a device outputting light having a wavelength of 400 to 700nm, preferably a wavelength of approximately 660nm, is used. It is noted that when an agent other than talaporfin sodium is used as a PDT agent, a wavelength of the light output by the light source 11 is not limited thereto.

As the light source 11, laser devices, such as a semiconductor laser device, light emitting diodes, or lamp light sources are used.

The excitation light output from the light source 11 enters a laser catheter 30 by the optical system 20.

The optical system 20 makes the excitation light emitted from the light source 11 incident into the laser catheter 30 via a polarizing beam splitter 21. Further, the optical system 20 extracts, from the laser catheter 30, fluorescence emitted from a PDT agent, which has been irradiated with the excitation light, and the excitation light returning from the laser catheter 30, and makes them incident into the fluorescence detection unit 12 and the return excitation light detection unit 13, respectively.

The optical system 20 includes the polarizing beam splitter 21, an optical fiber 22, a dichroic mirror 23, and a band pass filter 24.

The excitation light from the light source 11 enters the laser catheter 30. A part of the excitation light from the light source 11 is reflected on a side end face of an optical fiber and a connector, not illustrated, connecting between the therapy-progress-level monitoring device 1 and the laser catheter 30, and a tip portion of the laser catheter 30, and enters the polarizing beam splitter 21 as return excitation light.

Further, fluorescence from the tip portion of the laser catheter 30 also enters the polarizing beam splitter 21.

The polarizing beam splitter 21 reflects light made incident from the laser catheter 30 and guides it into the dichroic mirror 23 via the optical fiber 22.

The dichroic mirror 23 reflects light with a wavelength of 680 to 690nm or less and transmits light with a wavelength of 680 to 690nm or more. By this feature, the dichroic mirror 23 transmits fluorescence having a peak wavelength of approximately 710nm and guides it to the fluorescence detection unit 12 via the band pass filter 24, and reflects return excitation light having a peak wavelength of approximately 663nm and guides it to the return excitation light detection unit 13.

The band pass filter 24 is a filter that transmits light having a wavelength of 710±2nm.

Talaporfin sodium is used as a PDT agent in the present embodiment, thus the dichroic mirror 23 and the band pass filter 24 in use are configured to transmit and reflect light having the wavelengths as described above, however, when an agent other than talaporfin sodium is used as a PDT agent, configurations of the dichroic mirror 23 and the band pass filter 24 are not limited thereto.

The fluorescence detection unit 12 and the return excitation light detection unit 13 comprise a known linear image sensor and detect fluorescence of a PDT agent made incident from the optical system 20 and return excitation light from the laser catheter 30, respectively. The fluorescence detection unit 12 and the return excitation light detection unit 13 supply the detected fluorescence and return excitation light to the control unit 14 as an electric signal.

The fluorescence detection unit 12 includes a detection element such as a silicon photodiode, an avalanche photodiode, a spectrometer, and a photomultiplier tube. A measuring sampling rate of the detection element is 10 to 1,000Hz and preferably 100 to 500Hz.

The control unit 14 controls each portion in the therapy-progress-level monitoring device 1.

The control unit 14 calculates fluorescence intensity and return excitation light intensity based on the electric signal obtained from the fluorescence detection unit 12 and the return excitation light detection unit 13, and then calculates a therapy depth in real time based on these intensities.

The control unit 14 also outputs a display command to the display unit 16 for displaying calculation results.

The storage unit 17 is a non-volatile memory, and is set in, for example, a flash memory, an HDD, and other solid memories. The control unit 14 associates the calculated fluorescence intensity and excitation light intensity, the therapy depth, and time information obtained from a time measuring portion not illustrated for measuring, for example, a passage time counted from a reference time, such as an irradiation start time of excitation light, with each other and stores them in the storage unit 17 as a temporal change.

The display unit 16 is a display device, which uses, for example, a liquid-crystal display device. Upon obtaining a display command from the control unit 14, the display unit 16 displays, for example, information on the therapy depth and the time information in real time on a display screen based on display information included in the display command.

The operation unit 15 receives a command by an input operation from a practitioner and outputs the received command to the control unit 14. Examples of such a command include a command to turn on/off the excitation light output from the light source 11 and a command to change the intensity thereof.

In the therapy-progress-level monitoring device 1, the laser catheter 30 is detachably connected via a connector not illustrated to a tip of a tube not illustrated, which is in turn connected to the therapy-progress-level monitoring device 1.

The laser catheter 30 comprises a known configuration and includes a catheter tube 31 having a cylindrical hollow shape, an optical fiber 32 inserted in the catheter tube 31 along a longitudinal direction, an optical window 33 optically connected to a tip of the optical fiber 32 on the outermost of the tip portion of the laser catheter 30, and a holding portion 34 for holding the optical fiber 32 and the optical window 33 to the catheter tube 31.

In the present embodiment, the laser catheter 30 in use has an external diameter of 7Fr. (diameter of 2.33mm) and a spot diameter of 1.4mm, however the laser catheter 30 is not limited thereto.

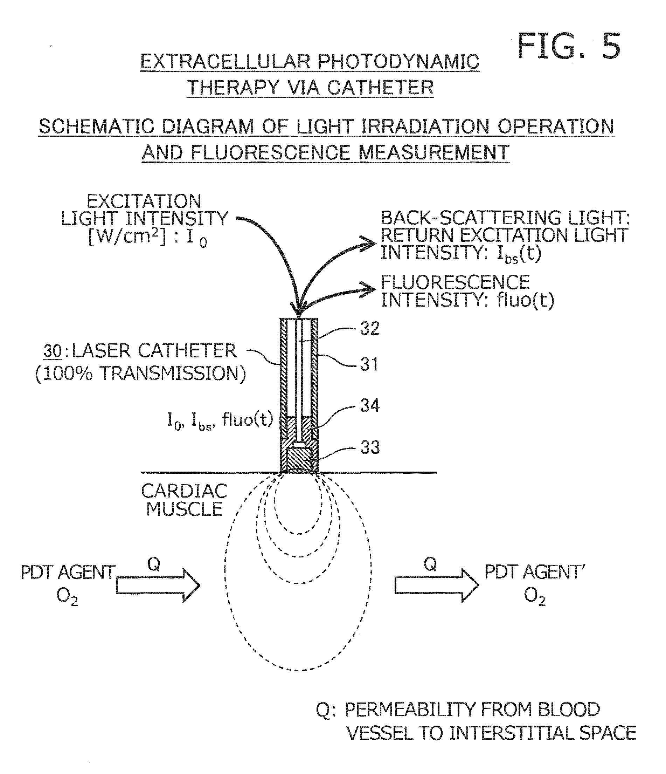

Here, Fig. 5 shows a schematic diagram illustrating a measurement of fluorescence and return excitation light when excitation light is irradiated from the laser catheter 30 to a therapy-target tissue.

As shown in Fig. 5, the optical fiber 32 transmits excitation light from the therapy-progress-level monitoring device 1 and emits the excitation light from its tip to the optical window 33. Further, the optical fiber 32 transmits fluorescence emitted from a PDT agent irradiated by the excitation light and return excitation light reflected from the tips, the inner surfaces, and the like of the optical window 33 and the optical fiber 32 to the therapy-progress-level monitoring device 1.

The optical window 33 is composed of a solid transparent material, such as a glass material, and transmits the excitation light emitted from the tip of the optical fiber 32 and irradiates the excitation light to a therapy-target tissue. Further, the optical window 33 condenses, on the tip of the optical fiber 32, the fluorescence emitted from the PDT agent irradiated by the excitation light and the return excitation light reflected from the tip of the optical window 33 and the like.

«Photodynamic therapy procedure and therapy-progress-level monitoring method»

The therapy-progress-level monitoring method of the present embodiment comprises: detecting, at a predetermined time during a detection period, fluorescence intensity produced when a photosensitive substance excited by a reaction between excitation light and the photosensitive substance is inactivated into a ground state, wherein the excitation light has a wavelength capable of exciting the photosensitive substance and is irradiated to the photosensitive substance distributed and sustainably supplied by vascular permeability in an extracellular fluid and/or a transcellular fluid in a living body; calculating in real time an indicator value estimating a volume or a depth of a therapy region by using a value of the fluorescence intensity at the predetermined time; and displaying the indicator value to a display unit in real time.

Hereinafter, the photodynamic therapy procedure and the therapy-progress-level monitoring method of the present embodiment are explained by taking a treatment of arrhythmia using photodynamic therapy as an example.

First, preparation for photodynamic therapy is performed.

In this example, the laser catheter 30 is inserted in the heart via the femoral vein or the jugular vein, not illustrated, of a patient by a practitioner such as a medical doctor. A tip portion of the laser catheter 30 is disposed in the vicinity of the pulmonary vein in an inner wall of the cardiac-muscle tissue of the left atrium.

Subsequently, a PDT agent is administered to the patient by the practitioner. In this example, further explanation is provided in a case where a necessary amount of the PDT agent for the therapy is administered to the patient at once by intravenous injection. The administered PDT agent is diffused in the interstitium or in the blood vessel.

After administering the PDT agent, a switch of the light source 11, not illustrated, is turned on by the practitioner after a predetermined period of time, for example, after several minutes to several tens of minutes, to initiate the emitting of excitation light from the light source 11. The excitation light is irradiated from the tip of the optical window 33 in the laser catheter 30.

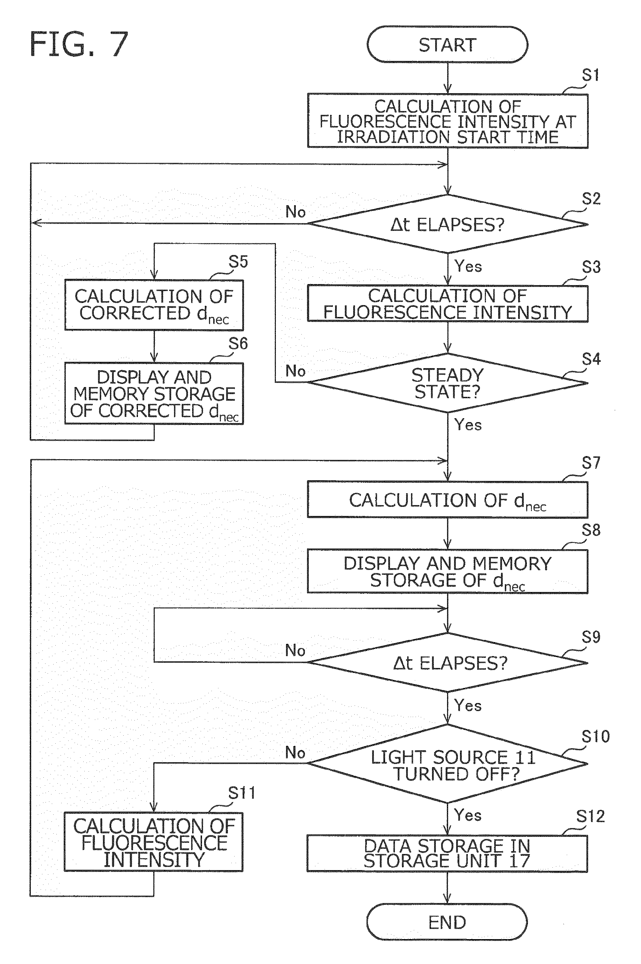

When the switch of the light source 11 is turned on, a timer not illustrated is turned on and a flowchart process in Fig. 7 is started. The flowchart process in Fig. 7 is controlled by the control unit 14. A time point when the switch of the light source 11 is turned on corresponds to a light irradiation start time.

First, in Step S1, fluorescence intensity detected by the fluorescence detection unit 12 at a time point when the switch of the light source 11 is turned on is calculated and stored in a storage not illustrated.

Then, in Step S2, it is determined whether a predetermined measurement time interval $\Delta t$ (sec) has elapsed since a previous calculation time point of the fluorescence intensity.

If $\Delta t$ (sec) has not elapsed (Step S2: No), Step S2 is repeated to determine whether the predetermined measurement time interval $\Delta t$ (sec) has elapsed. $\Delta t$ (sec) is set to a very short time, for example, about 0.05 sec to 0.5 sec.

If $\Delta t$ (sec) has elapsed (Step S2: Yes), it is considered that the predetermined measurement time interval has elapsed, so that the intensity of fluorescence is calculated at the time when the detection is made by the fluorescence detection unit 12 and stored in the storage not illustrated in Step S3.

Next, in Step S4, it is determined whether a fluorescence intensity waveform, drawn by plotting the fluorescence intensity calculated in Step S3 in a graph, has reached to a steady state.

When a difference of the fluorescence intensity between the present and the last time is equal to or more than a predetermined value, it is determined that the steady state is not reached, while when a difference is smaller than the predetermined value, it is determined that the steady state is reached.

If the fluorescence intensity waveform has not reached to the steady state (Step S4: No), a corrected estimated therapy depth $d_{nec}$ is calculated in Step S5. The corrected estimated therapy depth $d_{nec}$ is calculated by the following procedure.

First, a light irradiation time indicating a time from the light irradiation start time to a current time is obtained from the timer not illustrated, then the return excitation light intensity detected by the return excitation light detection unit 13 at the current time is calculated. Together with these calculations, the fluorescence intensity calculated in Step S3 or Step S8 is used to calculate a PBI by the following formula (1).

[Mathematical formula 1]

$$PBI = (fluo(0) - fluo(t_d))t_d \qquad \text{(formula 1)}$$

It is noted that, in formula 1, the following abbreviations are used:

fluo(t): return fluorescence intensity at a time point t
$t_d$: light irradiation time [s]

Next, this PBI is corrected by the correction methods that utilize a temporally changing fluorescence intensity waveform itself or the correction methods that utilize physiological information on a tissue and the like obtained by other methods, as described above, to obtain a corrected PBI.

Subsequently, the corrected estimated therapy depth $d_{ncc}$ is calculated by the following formula 3 using this corrected PBI.

[Mathematical formula 3]

$$d_{nec} = \frac{1}{\mu_{eff}} \ln\left(\frac{k(t)I_0 correctedPBI}{E}\right)$$

$$= \frac{1}{\mu_{eff}}\left\{\ln(correctedPBI) + \ln\left(\frac{k(t)I_0}{E}\right)\right\} \qquad \text{(formula 3)}$$

It is noted that, in formula 3, the following abbreviations are used:

$d_{nec}$: damage depth [mm]
$\mu_{eff}$: attenuation coefficient [/mm]
$k(t)$: constant that varies with time $1/\{fluo(0)-fluo(t)\}$
$I_0$: light intensity on tissue surface [mW]
E: light energy at given depth [J]

Next, in Step S6, data of the corrected estimated therapy depth $d_{ncc}$ calculated in Step S5 is sent to the display unit 16 as well as stored in the storage not illustrated.

With Step S6, the corrected estimated therapy depth $d_{ncc}$ is displayed in the display unit 16 in real time for allowing of visual recognition by a practitioner.

In this step, the corrected estimated therapy depth $d_{ncc}$ is displayed not as an absolute depth of damages caused to a tissue by photodynamic therapy, but as an indicator value thereof expressed as a relative value. Example of such an indicator value include an integer or a number expressed as decimals to approximately one or two places, such as "3" and "5.3".

Next, in Step S2, it is determined whether the predetermined measurement time interval $\Delta t$ (sec) has elapsed.

That is, the corrected estimated therapy depth $d_{nec}$ is calculated and displayed, instead of the estimated therapy depth $d_{nec}$, until the fluorescence intensity waveform reaches to the steady state.

Alternatively, it may be that, instead of calculating and displaying the corrected estimated therapy depth $d_{ncc}$ in Steps S5 and S6, neither the estimated therapy depth $d_{ncc}$ nor the corrected estimated therapy depth $d_{nec}$ is calculated or displayed until the steady state is reached by returning from Step S4 to Step S2.

Further Steps S4 to S6 may be skipped. When Steps S4 to S6 are skipped, the estimated therapy depth $d_{nec}$ is calculated and displayed immediately after the start of the light irradiation.

If the fluorescence intensity waveform reaches to the steady state (Step S4: Yes), a light irradiation time indicating a time from a light irradiation start time to a current time is obtained from the timer not illustrated in Step S7, then the return excitation light intensity detected by the return excitation light detection unit 13 at the current time is calculated. Together with these calculations, the fluorescence intensity calculated in Step S3 or Step S11 is used to calculate the estimated therapy depth $d_{nec}$ by the following formulas (1) and (2).
[Mathematical formula 1]

$$PBI = (fluo(0) - fluo(t_d))t_d \qquad \text{(formula 1)}$$

It is noted that, in formula 1, the following abbreviations are used:

$fluo(t)$; return fluorescence intensity at a time point t
$t_d$: light irradiation time [s]

[Mathematical formula 2]

$$d_{nec} = \frac{1}{\mu_{eff}} \ln\left(\frac{k(t)I_0 PBI}{E}\right)$$

$$= \frac{1}{\mu_{eff}}\left\{\ln(PBI) + \ln\left(\frac{k(t)I_0}{E}\right)\right\} \qquad \text{(formula 2)}$$

It is noted that, in formula 2, the following abbreviations are used:

$d_{nec}$: damage depth [mm]
$\mu_{efT}$: attenuation coefficient [/mm]
k(t): constant that varies with time 1/{fluo(0)-fluo(t)}
$I_0$: light intensity on tissue surface [mW]
E: light energy at given depth [J]

Next, in Step S8, data of the estimated therapy depth $d_{nec}$ calculated in Step S7 is sent to the display unit 16 as well as stored in the storage not illustrated.

With Step S8, the estimated therapy depth $d_{nec}$ is displayed in the display unit 16 in real time for allowing of visual recognition by a practitioner

In this step, the estimated therapy depth $d_{ncc}$ is displayed not as an absolute depth of damages caused to a tissue by photodynamic therapy, but as an indicator value thereof expressed as a relative value. Example of such an indicator value include an integer or a number expressed as decimals to approximately one or two places, such as "3" and "5.3".

In Steps S7 and S8, the estimated therapy depth $d_{nec}$ is calculated, displayed and stored in the storage. However, in Step S7, instead of the estimated therapy depth $d_{nec}$, the corrected estimated therapy depth $d_{nec}$ may be calculated using a corrected PBI corrected by the correction methods that utilize physiological information on a tissue and the like obtained by other methods, and then the corrected estimated therapy depth $d_{nec}$ may be displayed and sorted in Step S8.

In this case, in Step S7, after a PBI is calculated by the above formula 1, this PBI is corrected by the correction methods that utilize physiological information on a tissue and the like obtained by other methods to obtain a corrected PBI, and then, the corrected estimated therapy depth $d_{nec}$ is calculated by the above formula 3.

Further, in this case, a process of Step S7 is different from that of Step S5, performed before the steady state, in that Step S7 calculates a corrected PBI by the correction methods that utilize physiological information on a tissue and the like obtained by other methods, while Step S5 calculates a corrected PBI by the correction methods that utilize a temporally changing fluorescence intensity waveform itself or the correction methods that utilize physiological information on a tissue and the like obtained by other methods.

Next, in Step S9, it is determined whether a predetermined measurement time interval $\Delta t$ (sec) has elapsed since a previous calculation time point of the fluorescence intensity.

If $\Delta t$ (sec) has not elapsed (Step S9: No), Step S9 is repeated to determine whether the predetermined measurement time interval $\Delta t$ (sec) has elapsed.

If $\Delta t$ (sec) has elapsed (Step S9: Yes), it is determined whether the switch of the light source 11 is turned off in Step S10.

If the switch of the light source 11 is not turned off (Step S10: No), according to the judgment of the practitioner, it is considered that photodynamic therapy is still continued, so that the fluorescence intensity is calculated at the time when the detection is made by the fluorescence detection unit 12 in Step S11 and stored in the storage not illustrated.

Subsequently, Step S7 is repeated, so that a light irradiation time indicating a time from a light irradiation start time to a current time is obtained from the timer not illustrated, then the return excitation light intensity detected by the return excitation light detection unit 13 at the current time is calculated. Together with these calculations, the fluorescence intensity calculated in Step S3 or Step S11 is used to calculate an estimated therapy depth $d_{nec}$ by the above formulas (1) and (2).

If the switch of the light source 11 is turned off (Step S10: Yes), according to the judgment of the practitioner, it is considered that photodynamic therapy is finished, so that data stored in the storage not illustrated is stored in the storage unit 17 by processing Step S1 and S8, thereby completing the flowchart process in Fig. 7.

[Example]

Hereinafter, the present invention will be further specifically described in the following example.

In the example, a relation between a PBI calculated by the above formulas (1) and (2) in extracellular photodynamic therapy via a catheter for the cardiac muscle and a measured depth of necrosis $D_{nec}$ is investigated in order to evaluate the feasibility of estimating the measured depth of necrosis $D_{nec}$ by using an estimated therapy depth $d_{nec}$ calculated by the above formulas (1) and (2).

In the example, dogs (weight: $12.4 \pm 0.8$kg, N=16) were subjected to intercostal thoracotomy to expose their hearts and 2.5 or 5.0mg/kg of a PDT agent, namely talaporfin sodium, was intravenously administered.

When 2.5mg/kg was administered, an average concentration of the agent in the blood plasma was $15.85\mu$g/ml (S.D. : 2.00), while when 5.0mg/kg was administered, an average concentration of the agent in the blood plasma was $36.16\mu$g/ml (S.D. : 7.99).

Fifteen minutes after the administration, light irradiation was performed to the ventricular muscle by the laser catheter 30, and fluorescence intensity and return excitation light intensity were measured in each condition. In this operation, a wavelength of the irradiation light was set to 663 $\pm$ 2nm, a spot diameter to 1.4mm, irradiation intensity to 5, 10, and 20W/cm$^2$, and irradiation time to 5, 10, and 20 sec.

One example of a time-dependent change of the fluorescence intensity, when the dose of the PDT agent, talaporfin sodium, was set to 2.5mg/kg, the irradiation intensity to 20W/cm$^2$, and the irradiation time to 10 sec, is shown in Fig. 6. As shown in Fig. 6, the return fluorescence was attenuated concurrently with the irradiation time.

Further, a PBI was calculated by the formula (1) using the fluorescence intensity and the return irradiation light intensity.

Further after the light irradiation was performed using the laser catheter 30, histopathological evaluation was performed one week after the operation.

In the histopathological evaluation, a cross section in a light irradiation direction was cut out and subjected to a Hematoxylin-Eosin staining. A measured depth of necrosis $D_{nec}$ is determined by a depth of solid damaged sites continuously extending from a tissue surface.

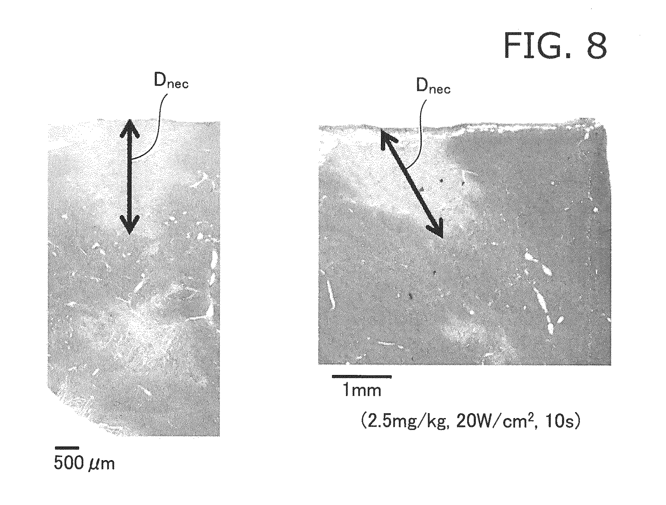

FIG. 8 shows an example of photograph showing a measurement of the measured depth of necrosis $D_{nec}$.

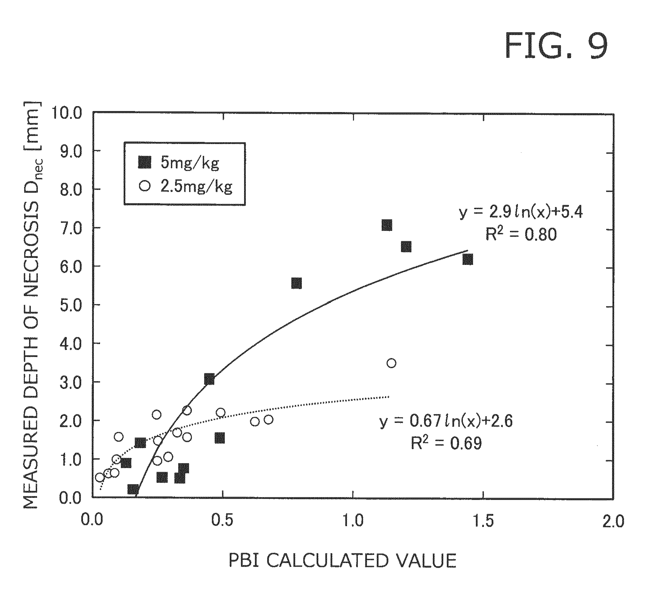

Fig. 9 shows a graph where the PBI calculated from the fluorescence intensity and the return excitation light intensity in each condition was plotted on a lateral axis and the measured depth of necrosis $D_{nec}$ in each condition, measured one weak after photodynamic therapy, was plotted on a longitudinal axis.

From Fig. 9, there was obtained a good correlation in a logarithm scale between the PBI and the measured depth of necrosis $D_{nec}$ in each condition. Therefore, it is proven that the measured depth of necrosis $D_{nec}$ can be estimated by the PBI.

Reference Numerals

| | |
|---|---|
| 1: | therapy-progress-level monitoring device |
| 11: | light source |
| 12: | fluorescence detection unit |
| 13: | return excitation light detection unit |
| 14: | control unit |
| 15: | operation unit |
| 16: | display unit |
| 17: | storage unit |
| 20: | optical system |
| 21: | polarizing beam splitter |
| 22: | optical fiber |
| 23: | dichroic mirror |
| 24: | band pass filter |
| 30: | laser catheter |
| 31: | catheter tube |
| 32: | optical fiber |
| 33: | optical window |
| 34: | holding portion |

Claims

1. A therapy-progress-level monitoring device that evaluates a progress level of photodynamic therapy for treating a therapy region of a living body using a photosensitive substance excited by light having a peak intensity in a predetermined range, the device comprising:

an irradiation unit configured to irradiate, with excitation light of a wavelength capable of exciting the photosensitive substance, the photosensitive substance distributed and sustainably supplied by vascular permeability in an extracellular fluid and/or a transcellular fluid of the living body;
a detection unit configured to detect, during a detection period, fluorescence intensity produced when the photosensitive substance excited by a reaction between the excitation light and the photosensitive substance is inactivated into a ground state;
a depth calculation unit configured to calculate in real time an indicator value estimating a volume or a depth of the therapy region by using a value of the fluorescence intensity during the detection period, received from the detection unit; and

an output unit configured to output in real time the indicator value calculated by the depth calculation unit to a display unit or a storage unit.

2. The therapy-progress-level monitoring device according to claim 1, wherein the detection unit further detects intensity of light having a wavelength of the excitation light irradiated by the irradiation unit as return excitation light intensity; and the depth calculation unit calculates the indicator value by using absolute values or relative values of the return excitation light intensity during the detection period, received from the detection unit, and the fluorescence intensity.

3. The therapy-progress-level monitoring device according to claim 1 or 2, wherein the detection period is a time period from a first light emission period from an irradiation start time of the excitation light until when the fluorescent intensity reaches a steady state to a second light emission period having the steady state continued from the first light emission period until when irradiation of the excitation light is ended, or a time period including only the second light emission period.

4. The therapy-progress-level monitoring device according to any of claims 1 to 3, wherein:

the irradiation unit comprises a laser catheter or an optical fiber; and

the depth calculation unit calculates a PBI (photosensitizer bleaching index), serving as a progress indicator for photodynamic therapy, by multiplying a difference between the fluorescence intensity at the irradiation start time of the excitation light and the fluorescence intensity at a specific time of irradiation by a time from the irradiation start time to the specific time of irradiation, and then calculates an indicator value estimating the volume or the depth of the therapy region using the PBI,

the specific time of irradiation being selected from a time range from the irradiation start time of the excitation light to a time when a contact state of a tip portion of the laser catheter or the optical fiber to a tissue of the living body changes.

5. The therapy-progress-level monitoring device according to claim 4, wherein the depth calculation unit comprises a correction unit configured to correct the indicator value, the correction unit executing at least one processing selected from a group consisting of:

a processing of subtracting the PBI in a final stage of the first light emission period from the PBI;

a processing of correcting the PBI in accordance with a state in progress in order for the PBI to reflect a photosensitizing reaction in a transition state of the first light emission period using ratios between the fluorescence intensity in the first light emission period and at the irradiation start time of the excitation light, and the fluorescence intensity in the second light emission period, when the specific time of irradiation is any time during the first light emission period;

a processing of multiplying the PBI by a detection value of the fluorescence intensity at the irradiation start time of the excitation light;

a processing of multiplying the PBI by a ration of the fluorescence intensity in the second light emission period to the fluorescence intensity at the irradiation start time of the excitation light;

a processing of dividing the PBI by the return excitation light intensity at the specific time of irradiation; and

a processing of correcting the PBI by using physiological information on a tissue obtained through measurement by other methods, information on systemic conditions of the living body obtained from other living-body monitoring devices, and known physiological numerical values related to a tissue according to the therapy region, to calculate a corrected PBI,

the depth calculation unit calculates an indicator value estimating the volume or the depth of the therapy region using the corrected PBI in real time or after the first light emission period, and

the output unit outputs the indicator value calculated by the depth calculation unit to a display unit or a storage unit in real time or after the first light emission period.

6. The therapy-progress-level monitoring device according to claim 5, wherein:

the depth calculation unit calculates an indicator value estimating the volume or the depth of the therapy region expressed as a relative value using a logarithm of the PBI or the corrected PBI; and

the output unit outputs the indicator value expressed as the relative value to the display unit or the storage unit.

**7.** The therapy-progress-level monitoring device according to any of claims 1 to 6, wherein the photosensitive substance is talaporfin sodium.

**8.** The therapy-progress-level monitoring device according to any of claims 1 to 7, wherein the excitation light is light having a wavelength of 400 to 700nm and light emitted from a laser beam, a light emitting diode, or a lump source.

**9.** The therapy-progress-level monitoring device according to any of claims 2 to 8, wherein the detection unit comprises a detection element selected from a group consisting of a silicon photodiode, an avalanche photodiode, a spectrometer, and a photomultiplier tube, and detects the fluorescence and the return excitation light at a sampling rate of 10 to 1,000Hz.

**10.** A therapy-progress-level monitoring device that evaluates a progress level of photodynamic therapy for treating a therapy region of a living body using a photosensitive substance excited by light having a peak intensity in a predetermined range, the device comprising:

a receiving unit configured to receive a value of fluorescence intensity detected by a detection unit configured to detect, during a detection period, the fluorescence intensity produced when the photosensitive substance is inactivated into a ground state after having being excited by a reaction between excitation light and the photosensitive substance, the excitation light having a wavelength enabling to excite the photosensitive substance and being irradiated to the photosensitive substance distributed and sustainably supplied by vascular permeability to an extracellular fluid and/or a transcellular fluid of the living body;
a depth calculation unit configured to calculate in real time an indicator value estimating a volume or a depth of the therapy region by using a received value of the fluorescence intensity during the detection period; and
an output unit configured to output the indicator value calculated by the depth calculation unit to a display unit or a storage unit for allowing real time displaying and storing.

**11.** A therapy-progress-level monitoring method that evaluates a progress level of photodynamic therapy for treating a therapy region of a living body using a photosensitive substance excited by light having a peak intensity in a predetermined range, the method comprising:

a receiving procedure configured to receive a value of fluorescence intensity detected by a detection unit configured to detect, at a predetermined time point during a detection period, the fluorescence intensity produced when the photosensitive substance is inactivated into a ground state after having being excited by a reaction between excitation light and the photosensitive substance, the excitation light having a wavelength enabling to excite the photosensitive substance and being irradiated to the photosensitive substance distributed and sustainably supplied by vascular permeability to an extracellular fluid and/or a transcellular fluid of the living body;
a depth calculation procedure configured to calculate in real time an indicator value estimating a volume or a depth of the therapy region by using a received value of the fluorescence intensity at the predetermined time point; and
an output procedure configured to output the indicator value calculated by the depth calculation unit to a display unit or a storage unit for allowing real time displaying and storing.

FIG. 1

FIG. 2

IRRADIATION $I_0$

IRRADIATION DOSE

$$\int_0^{t_d} {}^1O_2 dt$$

Depth

PRODUCTION

$^3O_2$

PDT AGENT

SUPPLY

REACTOR:
EXTRACELLULAR
PHOTOSENSITIZING
REACTION IN
INTERSTITIAL SPACE

DESTRUCTION
OF PDT AGENT-
BOUND PROTEIN

$^1O_2 \rightarrow {}^3O_2$

EFFECT OF THERAPY

AGENT BLEACHING
$\propto -\Delta\, \text{fluo}(t)$

fluo(t)

fluo(t): PDT AGENT FLUORESCENCE
$t_d$: IRRADIATION TIME

FIG. 3

FIG. 4

EP 2 997 999 A1

# FIG. 5

## EXTRACELLULAR PHOTODYNAMIC THERAPY VIA CATHETER

## SCHEMATIC DIAGRAM OF LIGHT IRRADIATION OPERATION AND FLUORESCENCE MEASUREMENT

EXCITATION LIGHT INTENSITY $[W/cm^2] : I_0$

BACK-SCATTERING LIGHT: RETURN EXCITATION LIGHT INTENSITY: $I_{bs}(t)$

FLUORESCENCE INTENSITY: fluo(t)

32
31
34
33

30: LASER CATHETER (100% TRANSMISSION)

$I_0, I_{bs}, fluo(t)$

CARDIAC MUSCLE

PDT AGENT $O_2$   Q $\rightarrow$

Q $\rightarrow$ PDT AGENT' $O_2$

Q: PERMEABILITY FROM BLOOD VESSEL TO INTERSTITIAL SPACE

FIG. 6

FLUORESCENCE INTENSITY AT LIGHT IRRADIATION START TIME

TIME LENGTH OF FIRST LIGHT EMISSION PERIOD

# FIG. 7

START

↓

CALCULATION OF FLUORESCENCE INTENSITY AT IRRADIATION START TIME — S1

↓

Δt ELAPSES? — S2
No → (loop back)
Yes ↓

CALCULATION OF FLUORESCENCE INTENSITY — S3

↓

STEADY STATE? — S4
No ↓

CALCULATION OF CORRECTED $d_{nec}$ — S5

↓

DISPLAY AND MEMORY STORAGE OF CORRECTED $d_{nec}$ — S6

STEADY STATE? Yes ↓

CALCULATION OF $d_{nec}$ — S7

↓

DISPLAY AND MEMORY STORAGE OF $d_{nec}$ — S8

↓

Δt ELAPSES? — S9
No → (loop back)
Yes ↓

LIGHT SOURCE 11 TURNED OFF? — S10
No ↓

CALCULATION OF FLUORESCENCE INTENSITY — S11

LIGHT SOURCE 11 TURNED OFF? Yes ↓

DATA STORAGE IN STORAGE UNIT 17 — S12

↓

END

FIG. 8

$D_{nec}$

$D_{nec}$

1mm

(2.5mg/kg, 20W/cm², 10s)

500 μm

FIG. 9

## INTERNATIONAL SEARCH REPORT

International application No.

PCT/JP2014/062566

**A. CLASSIFICATION OF SUBJECT MATTER**
*A61N5/067*(2006.01)i, *A61N5/06*(2006.01)i

According to International Patent Classification (IPC) or to both national classification and IPC

**B. FIELDS SEARCHED**

Minimum documentation searched (classification system followed by classification symbols)
A61N5/067, A61N5/06

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2010-536475 A (Cardiac Pacemakers, Inc.), 02 December 2010 (02.12.2010), entire text; all drawings & US 2009/0054883 A1 & WO 2009/025826 A1 | 1-10 |
| A | WO 2011/114651 A1 (Sony Corp.), 22 September 2011 (22.09.2011), entire text; all drawings & JP 2011-189020 A & US 2013/0172697 A1 & EP 2548614 A1 | 1-10 |
| A | WO 2011/114653 A1 (Sony Corp.), 22 September 2011 (22.09.2011), entire text; all drawings & JP 2011-212423 A & US 2013/0123642 A1 & EP 2548616 A1 | 1-10 |

☒ Further documents are listed in the continuation of Box C.          ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 July, 2014 (25.07.14) | 05 August, 2014 (05.08.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

### INTERNATIONAL SEARCH REPORT

| International application No. |
|---|
| PCT/JP2014/062566 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2012/076631 A1  (SPECTRACURE AB), 14 June 2012 (14.06.2012), entire text; all drawings (Family: none) | 1-10 |

Form PCT/ISA/210 (continuation of second sheet) (July 2009)

<div align="center">**INTERNATIONAL SEARCH REPORT**</div>

| International application No. |
| --- |
| PCT/JP2014/062566 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 11
    because they relate to subject matter not required to be searched by this Authority, namely:
    Claim 11 pertains to [a method for treatment of the human body by therapy].

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

    ☐ The additional search fees were accompanied by the applicant's protest but the applicable protest fee was not paid within the time limit specified in the invitation.

    ☐ No protest accompanied the payment of additional search fees.

Form PCT/ISA/210 (continuation of first sheet (2)) (July 2009)

**EP 2 997 999 A1**

**Patent documents cited in the description**

- WO 2008066206 A
- US 6128525 A
- JP 6080671 A
- JP 2961074 B